# EUROPEAN PATENT APPLICATION

(11) **EP 1 541 691 A1**
(43) Date of publication of application: **15.06.2005**
(21) Application number: 03741530.4
(22) Date of filing: 22.07.2003
(51) Int. Cl.: C12P 13/12, C07C 319/28, C07C 323/58

(54) **PROCESS FOR THE PRODUCTION OF METHIONINE**

(30) Priority: 23.07.2002 JP 2002214508
(71) Applicant: NIPPON SODA CO., LTD., Chiyoda-ku, Tokyo 100-8165 (JP)
(72) Inventor: KOBAYASHI, Yoichi, c/o NIPPON SODA CO., LTD, Odawara-shi, Kanagawa 250-0216 (JP); ONO, Ippei, c/o NIPPON SODA CO., LTD, Nakakubiki-gun, Niigata 949-2302 (JP); HAYAKAWA, Koichi, c/o NIPPON SODA CO., LTD, Odawara-shi, Kanagawa 250-0216 (JP); MIZUI, Yoshinori, c/o NIPPON SODA CO., LTD, Odawara-shi, Kanagawa 250-0216 (JP); ISHIKAWA, Takahiro, c/o NIPPON SODA CO., LTD, Nakakubiki-gun, Niigata 949-2302 (JP)
(74) Representative: Williams, Aylsa
(86) International application number: PCT/JP2003/009268
(87) International publication number: WO 2004/009829

(57) **Abstract**

The present invention relates to a practical method for producing methionine, wherein when a substance capable of producing methionine through hydrolysis is used as raw material and converted into methionine by using a biocatalyst, the biocatalyst is repeatedly usable and the accumulation amount of methionine dissolved in the reaction solution is increased, and methionine is obtained in a solid form from the reaction solution. Specifically, the present invention relates to a method for producing methionine comprising the following steps: a first step of converting raw material capable of producing methionine through hydrolysis of 2-amino-4-methylthiobutyronitrile, and 2-amino-4-methylthiobutanamide or the like in an aqueous ammonia solution, into methionine-containing aqueous ammonia solution, with a biocatalyst through hydrolysis, a second step of separating the biocatalyst from the methionine-containing aqueous ammonia solution obtained in the first step, and a third step of distilling ammonia out of the methionine-containing aqueous ammonia solution separated in the second step to deposit and separate crystals of methionine.

## Description

### Technical Field

The present invention relates to a method for producing crystals of methionine used as pharmaceuticals and feed additives. The provision of methionine in a solid form (crystal form) is required especially in feed additive market.

### Background Art

As methods for producing 2-amino acid by using the nitrile-hydrolyzing activity of a biocatalyst, methods using 2-aminonitrile or 2-aminoamide as raw material (Japanese Patent Publication No. 58-15120, Domestic republication of PCT international publication No. 63-500004, Japanese Laid-Open Patent Application No. 2-31694, Domestic republication of PCT international publication No. 3-500484, Japanese Patent Publication No. 3-16118, WO 02/08439, Japanese Laid-Open Patent Application No. 2002-34593, andUSPatentNo. 6417395) are known. A method using cyanhydrin as raw material (Japanese Laid-Open Patent Application No. 9-140391) is known as a method for producing 2-amino acid as well. However, none of them discloses a practical method for separating and purifying efficiently 2-amino acid having a solid form with low water solubility such as methionine from a biocatalyst.

Further, methods for separating ammonia produced in reaction solution, in microbiological production of glycine, from a glycinonitrile obtained from a reaction of formaldehyde, prussic acid and ammonia are known (Japanese Laid-Open Patent Application Nos. 2001-258586, 2001-299377, 2001-340096, and 2001-340097). The ammonia separation in these methods is performed in order to limit the pH increase caused by the reaction, by separating ammonia produced through hydrolysis of nitrile. Further as the water solubility is high (25 g/100g·H₂O at 25° C), glycine is not crystallized by distilling out ammonia.

The object of the present invention is to provide a practical method for producing methionine, wherein when 2-amino-4-methylthiobutyronitrile, 2-amino-4-methylthiobutanamide or the like which is a substance capable of producing methionine through hydrolysis is used as rawmaterial and converted into methionine by using a biocatalyst, the biocatalyst can be used repeatedly, the accumulation amount of methionine dissolved in the reaction solution is increased, and the methionine can be separated from the reaction solution in a solid form.

The present inventors have found previously nitrilase-producing bacteria Arthrobacter sp. NSSC 104 (FERM BP-5829) and Arthrobacter sp. NSSC 204 (FERM BP-7662), which can stably keep high nitrilase activity. In the process of investigating the manufacturing technology to obtain methionine by using nitrilase-producing bacteria as biocatalysts, through hydrolysis of 2-amino-4-methylthiobutyronitrile, as methionine has low water solubility (3.38 g/100g·H₂O at 25°C, 6.07 g/100g·H₂O at 50° C), crystals of methionine begin to deposit soon after the initiation of the reaction, aggregate with the biocatalyst, so it becomes difficult that the three components, i. e. , deposited crystals of methionine, biocatalyst and reaction solution separate effectively, and therefore, it became difficult to recycle the biocatalyst at the high yield. On the other hand, although it is possible to separate and recover the biocatalysts by maintaining the condition in which methionine is dissolved in the reaction solution (water) without deposit during the reaction, it was revealed that this method provides producing technique with so low productivity that it comprises poor practical utility, because accumulation concentration of methionine needs to be maintained at low concentration of less than 5% due to low water-solubility of methionine in this case, hence accumulation amount of methionine dissolved in the reaction solution can not be increased.

The above-described problem occurs similarly when obtaining methionine through hydrolysis of 2-amino-4-methylthiobutanamide by using amidase-producing bacteria as a biocatalyst.

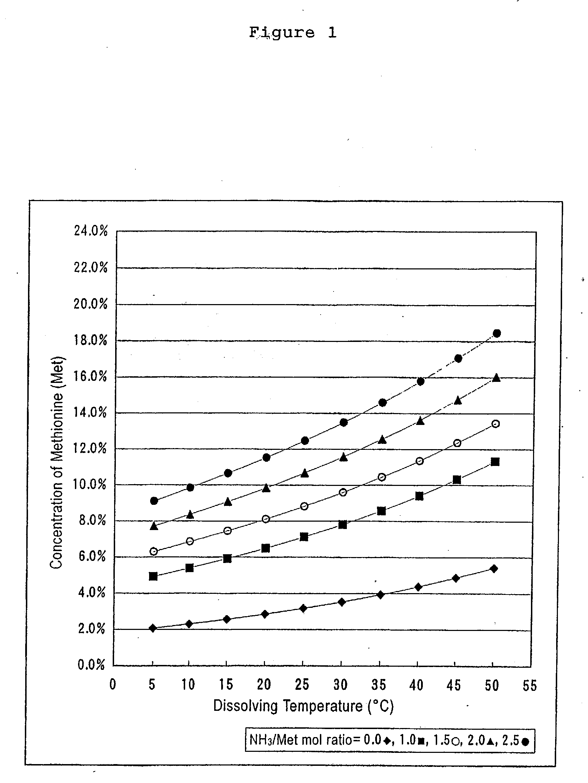

As a result of intensive search to solve the aforementioned objects of the present invention simultaneously, the present inventors assumed that ammonia should exist in the reaction solution to increase the accumulation amount of methionine dissolved in the reaction solution, and prepared 5 types of solutions, having a mole ratio of 0, 1, 1.5, 2, 2.5 ammonia to methionine, conducted preliminary experiments to examine the solubility of methionine at 5-50° C. From the results shown in Fig. 1, the present inventors have found that the solubility of methionine increases when ammonia is present at an excessive amount in mole ratio to methionine in the reaction solution, and devised a system to recover 100% of bacteria catalyst, by accumulating 5-30 wt% of methionine in the reaction solution in dissolved condition. About 20 wt% of methionine also can be dissolved in water by addition of inorganic alkali such as NaOH and KOH, while in this case it is required to neutralize with the use of acid to obtain a product in the form of salt-free solid of methionine, leading to a problem that an additional operation to remove a lot of inorganic salt waste generated from the neutralization is required.

In addition, separation and recovery of salt-free solid of methionine can be performed easily by distilling ammonia out of an aqueous ammonia solution containing methionine discharged out of hydrolysis reaction bath after separation of biocatalyst (methionine-containing aqueous ammonia solution), and by taking deposited crystals of methionine. As mother liquor from which crystals of methionine are separated and recovered, contains a small amount of remaining raw material of 2-amino-4-methylthiobutyronitrile, 2-amino-4-methylthiobutanamide, methionine, and ammonia, it is desirable to recycle it for hydrolysis reaction bath, which results in the completion of the process without waste generation. The present invention has thus been completed according to the findings mentioned above.

### Disclosure of the Invention

The present invention relates to:
[1] a method for producing methionine comprising the following steps: (1) a first step of converting raw material capable of producing methionine through hydrolysis into a form of methionine-containing aqueous ammonia solution through hydrolysis in an aqueous ammonia solution with a biocatalyst having hydrolyzing activity, (2) a second step of separating the biocatalyst from the methionine-containing aqueous ammonia solution obtained in the first step, and (3) a third step of distilling ammonia out of the methionine-containing aqueous ammonia solution separated in the second step to deposit and separate crystals of methionine ("1");
[2] the method for producing methionine according to " 1 " , wherein 2-amino-4-methylthiobutyronitrile as raw material is hydrolyzed in an aqueous ammonia solution with a biocatalyst having nitrile-hydrolyzing activity ("2");
[3] the method for producing methionine according to "1", wherein 2-amino-4-methylthiobutanamide as raw material is hydrolyzed in an aqueous ammonia solution with a biocatalyst having amide-hydrolyzing activity ("3");
[4] the method for producing methionine according to any one of "1" to "3", wherein an aqueous solution containing 1.5 to 10 fold equivalent amount of ammonia corresponding to that of methionine in the methionine-containing aqueous ammonia solution obtained in the first step, is used as aqueous ammonia solution ("4");
[5] the method for producing methionine according to any one of "1" to "4", wherein the concentration of methionine in the methionine-containing aqueous ammonia solution obtained in the first step is 5-30 wt% ("5");
[6] the method for producing methionine according to any one of "1" to "5", wherein the biocatalyst is reused ("6");
[7] the method for producing methionine according to any one of "1" to "6", wherein immobilized bacteria are used as the biocatalysts ("7");
[8] the method for producing methionine according to any one of "1" to "7", wherein the distilled ammonia and the mother liquor from which crystals of methionin are separated and recovered are reused for hydrolysis reaction ("8"); and
[9] the method for producing methionine according to any one of "1" to "8", wherein the first step is performed under pressurization ("9").

### Brief Description of Drawings

Fig. 1 is a figure showing the results of the examination of solubility of methionine at 5-50° C in 5 types of solutions, wherein the mole ratio of ammonia to methionine is 0, 1, 1.5, 2, and 2.5.
Fig. 2 is a figure showing the outline of a process without waste generation in the method for producing methionine of the present invention.

### Best Mode of Carrying Out the Invention

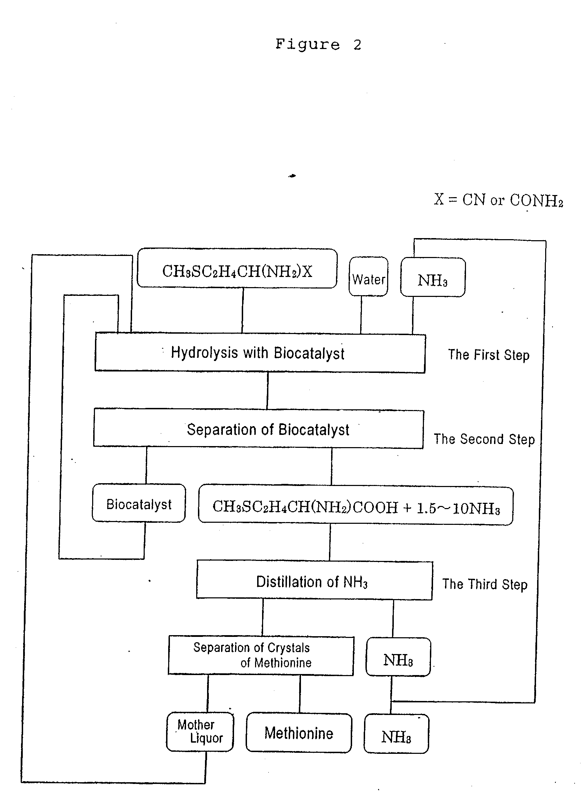

The method for producing methionine of the present invention is not especially limited as long as it comprises the following steps: (1) a first step of converting raw material capable of producing methionine through hydrolysis into a form of methionine-containing aqueous ammonia solution through hydrolysis in an aqueous ammonia solution with a biocatalyst having hydrolyzing activity, preferably with a biocatalyst having nitrile-hydrolyzing activity to hydrolyze 2-amino-4-methylthiobutyronitrile as raw material in an aqueous ammonia solution, or with a biocatalyst having amide-hydrolyzing activity to hydrolyze 2-amino-4-methylthiobutanamide as raw material in an aqueous ammonia solution, (2) a second step of separating the biocatalyst from the methionine-containing aqueous ammonia solution obtained in the first step, and (3) a third step of distilling ammonia out of the methionine-containing aqueous ammonia solution separated in the second step to deposit and separate crystals of methionine. However, a process without waste generation which reduces the biocatalyst or the like is preferable, e.g. as shown in Fig. 2.

As for rawmaterial capable of producing methionine through hydrolysis other than the aforementioned 2-amino-4-methylthiobutyronitrile and 2-amino-4-methylthiobutanamide, 2-amino-4-methylthiobutanamide lower alkyl ester, methylthioethylhydantoin, methylthioethylhydantoic acid, methylthioethylhydantoic acid amide and the like can be exemplified.

As for a biocatalyst used in the first step, wherein 2-amino-4-methylthiobutyronitrile is converted into methionine-containing aqueous ammonia solution through hydrolysis in an aqueous ammonia solution with a biocatalyst having nitrile-hydrolyzing activity, it is not especially limited as long as it is a biocatalyst e. g. microorganisms having nitrile-hydrolyzing activity in a solution such as an aqueous ammonia solution. Microorganisms belonging to genus Arthrobacter, genus Variovorax and so on can be exemplified, among which Arthrobacter sp. NSSC 104, Arthrobacter sp. NSSC 204, and Variovorax paradoxus IAM 12374 can be preferably exemplified as such biocatalyst.

Arthrobacter NSSC 104 was deposited with accession No. FERM BP-5829 at International Patent Organism Depositary, National Institute of Advanced Industrial Science and Technology, (Tsukuba Central 6, 1-1-1 Higashi, Tsukuba, Ibaraki, Japan) on February 6, 1996, and mycological nature thereof is described in WO No. 97/32030. Arthrobacter NSSC 204 was similarly deposited with accession No. FERMBP-7662 at International Patent Organism Depositary, National Institute of Advanced Industrial Science and Technology, (Tsukuba Central 6, 1-1-1 Higashi,-Tsukuba, Ibaraki, Japan) on June 22, 2000, and mycological nature thereof is described in WO No. 02/08439. In addition, Variovorax paradoxus IAM 12374 is easily available from Institute of Molecular and Cellular Biosciences of University of Tokyo, and mycological nature thereof is described in International Journal of Systematic Bacteriology, vol.41, 445-450, 1991.

As for a biocatalyst used in the first step, wherein 2-amino-4-methylthiobutanamide is converted into methionine-containing aqueous ammonia solution through hydrolysis in an aqueous ammonia solution with a biocatalyst having amide-hydrolyzing activity, it is not especially limited as long as it is a biocatalyst e.g. microorganisms having amide-hydrolyzing activity in solution such as aqueous ammonia solution, and Rhodococcus rhodochrous IFO 15564 can be preferably exemplified as the biocatalyst.

Rhodococcus rhodochrous IFO 15564 is easily available from National Institute of Technology and Evaluation Biological Resource Center (NBRC), and mycological nature thereof is disclosed in Tetrahedron Letters Vol. 32, 1343-1346.

The culture of these microorganisms is conducted in normal media containing carbon source, nitrogen source and inorganic ion, which can be assimilated by microorganisms and enzyme inducer, and further organic nutrient if required. As for an enzyme inducer, nitrile compounds such as isobutyronitrile, 2-aminobenzonitrile, and cyclic amide compounds such as ε-caprolactam are used, and especially 2-aminobenzonitrile is preferable. As for carbon source, carbon hydrate such as glucose, alcohols such as ethanol, organic acid or the like are suitably used. As for nitrogen source, amino acid, nitrate salt, ammonium salt and the like are used. As for_inorganic ion, phosphate ion, potassium ion, magnesium ion, sulfate ion, ferric ion and the like are used as required. As for organic nutrient source, vitamins, amino acid and the like and corn steep liquor containing these, yeast extract, polypeptone, meat extract, and so on are suitably used. The culture may be conducted under aerobic condition by controlling to maintain the suitable range of pH of 6-9 and temperature of 25-37°C.

As for a biocatalyst used in the present invention, bacteria cultured as mentioned above, immobilized bacteria prepared therefrom, and bacteria-treated product such as crude enzyme or immobilized enzyme can be exemplified. Commonly-conducted immobilizing technique such as carrier binding method, entrapment or the like can be applied in immobilizing bacteria or enzyme. In preparing enzyme or crude enzyme, bacteria are broken with ultrasonic wave, high-pressure homogenizer or the like, and then commonly-conducted technique of enzyme purification such as ammonium sulfate precipitation, chromatography or the like can be applied to the resultant. Meanwhile, as the biocatalyst used in the reaction such as bacteria can be repeatedly used in hydrolysis reaction without practical reduction of activity thereof, it is preferable to reuse it.

Hydrolysis reaction with the biocatalyst is performed with raw material capable of producing methionine by acting the aforementioned biocatalyst in an aqueous solvent containing ammonia through hydrolysis of 2-amino-4-methylthiobutyronitrile, 2-amino-4-methylthiobutanamide and the like. The biocatalyst is usually used at a concentration of 0.1-10 wt%, preferably 1-6 wt % converting to dry weight. Further, raw material such as 2-amino-4-methylthiobutyronitrile, 2-amino-4-methylthiobutanamide or the like is used for the reaction at a concentration of 0.01-50 wt%, and can be successively or continuously added during the reaction if necessary. Furthermore, as for an aqueous solvent containing ammonia, it may be an aqueous solvent containing organic solvent having aqueous ammonia as major component, which may contain organic base such as amine, organic acid or inorganic acid. Ammonia is used in an aqueous solution at a concentration of 0.5-30 wt%, preferably 0.8-10 wt%, and aqueous solution containing 1.5 to 10 fold equivalent amount of ammonia corresponding to that of accumulated concentration of methionine in the methionine-containing aqueous ammonia solution can be used. In addition, hydrolysis reaction can also be performed under pressurization in order to enhance the dissolved amount of ammonia and increase the accumulation amount of methionine dissolved in the reaction solution.

In the second step wherein the biocatalyst is separated from the methionine-containing aqueous ammonia solution obtained in the above first step, the methionine-containing aqueous ammonia solution can be excreted out of the reaction system by being separated from the biocatalyst, or the biocatalyst can be excreted out of the reaction system by being separated from the methionine-containing aqueous ammonia solution. As for such a method for separating the methionine-containing aqueous ammonia solution from the biocatalyst after the hydrolysis reaction, it is not especially limited as long as it is a known method for solid-liquid separation, and it can be performed by filtration, centrifugation, ultrafiltration and the like. The recovered biocatalyst can be repeatedly used for hydrolysis reaction as aforementioned. Further, separation of the biocatalyst from methionine-containing aqueous ammonia solution when immobilized bacteria or immobilized enzyme is used, specific means for solid-liquid separation is not required. Discharge of immobilized biocatalyst from reaction bath may be prevented by setting a simple coarse-meshed filter such as strainer to a drain outlet of the reaction bath.

In the third step wherein ammonia is distilled out of the methionine-containing aqueous ammonia solution separated in the above second step to deposit and separate crystals of methionine, distilling ammonia out of the methionine-containing aqueous ammonia solution is conducted through deaeration under high or reduced pressure, or heating distillation, and distilling out a certain amount of ammonia excessive to methionine allows crystallization of methionine. Distilled ammonia can be reused in hydrolysis reaction except the equimol amount of methionine. Excluded ammoniawhich cannot be reused, can be used to synthesize raw material such as 2-amino-4-methylthyobutyronitrile and 2-amino-4-methylthiobutanamide. As stated above, crystallized methionine can be recovered as a solid product by using a solid-liquid separator such as filter/centrifugal separation. After crystals of methionine are recovered and separated, the mother liquor can be recycled for hydrolysis reaction as it contains small amount of raw material such as remaining 2-amino-4-methylthiobutyronitrile and 2-amino-4-methylthiobutanamide, methionine, ammonia and the like.

Methionine produced in the present invention can be obtained as a D-type, L-type, or racemate methionine depending on the optical selectivity of the used biocatalyst. Crystals of methionine produced and separated can be subjected to further purification or adjustment of particle size as required.

The present invention will be described more specifically by referring to the examples in the following, but the present invention is not limited to these examples.

### Example 1. (Production of DL-methionine with NSSC 204 strain) (Culture of NSSC 204 strain)

Two ml of medium containing 0.5% of yeast extract, 0.5% of glucose, 0.1% of dipotassium hydrogenphosphate, 0.1% of potassium dihydrogenphosphate, 0.1 % of sodium chloride, 0.02% of magnesium sulfate heptahydrate, 0.001% of ferrous sulfate, and 0.03% of 2-aminobenzonitrile was poured into a test tube and sterilized for 20 min at 121° C. After 1 platinum loop of Arthrobacter NSSC 204 strain was inoculated into the test tube, the mixture was cultured with shaking overnight at 33°C, to prepare the pre-culture. Subsequently, 20 ml of medium of pH 7.2 (adjusted with 2N of caustic soda) containing 2.0% of corn steep liquor (filtered and sterilized), 1.0% of sucrose (sterilized for 20 min at 121° C), 0.03% of 2-aminobenzonitrile (sterilized for 20 min at 121° C) was poured into a 100 ml-Erlenmyer flask with a baffle, reinoculated with 0.2 ml of the above-described pre-culture, and was further cultured with shaking for 4 days at 33°C.

### (Production of DL-methionine)

Obtained culture solution of Arthrobacter NSSC 204 strain was subjected to centrifugation, then washed with ion-exchanged water, and the resultant was suspended in an aqueous solution (pH 11.2) containing _ 133 mM of 2-amino-4-methylthiobutyronitrile and 25 mM of 1,3-diaminopropane so that the concentration of dried bacteria becomes 0.1% (w/w) and subjected to hydrolysis reaction by shaking slowly at 35°C. Four hours after the addition, the bacteria was removed by centrifugation and the concentration of methionine contained in the remaining reaction solution was determined by using high performance liquid chromatography (column: TSKgel ODS-80TM, carrier: ethanol/water/trifluoroacetic acid=5/95/0.04) and accumulation of 125 mM of DL-methionine was confirmed as a result.

### Example 2. (Continuous production of DL-methionine by NSSC 204 strain.)

Obtained culture solution of Arthrobacter NSSC 204 strain in Example 1 was subjected to centrifugation, then washed with ion-exchanged water, and the resultant was suspended in an aqueous solution (pH 9.5) containing 10% (w/w) of DL-methionine and 2.28% of ammonia so that the concentration as dried bacteria becomes 2% (w/w). 300 g of the bacteria-suspension was poured into a 500 ml-three-necked flask and incubated at 30°C, and 2-amino-4-methylthiobutyronitrile was sequentially added by stirring at a speed of 5.5 g/h. On the other hand, microfiltrationmembrane (Asahi kasei Co. LTD. , microza PMP-003) was used to filter the bacteria sequentially, and the reaction filtrate was recovered at a speed of about 54 g/h. At that time, 1.14% (w/w) of aqueous ammonia solution in equal amount of the reaction filtrate, was sequentially replenished by using a pump coupled with a liquid level-sensor, so that the liquid level in the reaction container did not reduce. The concentration of DL-methionine of recovered reaction filtrate was measured every hour by using high performance liquid chromatography (column: TSKgel ODS-80TM, carrier: ethanol/water/trifluoroacetic acid=5/95/0.04) and the speed at which the reaction filtrate was recovered was controlled to maintain the concentration of the reaction filtrate at 10% (w/w) . The concentration of 2-amino-4-methylthiobutyronitrile contained in the reaction filtrate increased gradually, reached 0.4% (w/w) at 8 h after the initiation of the reaction, and the concentration was maintained for 8 days, during which the production speed of methionine was 5.36 g/h.

### Example 3. (Continuous production of DL-methionine by immobilized NSSC 204 strain)

### (Immobilization of NSSC 204 strain)

Culture solution of Arthrobacter NSSC sp. 204 obtained in Example 1 was subjected to centrifugation, then washed with ion-exchanged water, and the resultant was suspended in 1% (w/w) of sodium alginate solution so that the concentration as dried bacteria becomes 10% (w/w). Subsequently, the suspension was dropped to 0.1 M of calcium chloride solution to produce immobilized bacteria-beads. A column having an inside diameter of 30 mm was filled with 225 g of obtained immobilized bacteria-beads, and the column was equilibrated by flowing 1 1 of aqueous solution (pH 9.5) containing 10% (w/w) DL-methionine, 2.28% (w/w) ammonia, 2.5 mM of 1,3-diaminopropane, and 10 mM of calcium chloride at a flow late of 0.21/h. Then, the beads were poured into a 500 ml-three-necked flask incubated at 30° C, the equilibrated aqueous solution was added to it so that the total amount became 300 g, and then 2-amino-4-methylthiobutyronitrile was continuously added to the resultant by stirring at a speed of 5.16 g/h. On the other hand, the reaction solution was recovered through a suction filter at a speed of about 50 g/h so that the immobilized beads might not be sucked out. At that time, 1.14% (w/w) of aqueous ammonia solution containing 20 mM of ethylenediamine and 10 mM of calcium chloride in equal amount of the reaction filtrate, was continuously replenished by using a pump coupled with a liquid level-sensor, so that the liquid level in the reaction container did not reduce. The concentration of DL-methionine of recovered reaction filtrate was measured every hour by using high performance liquid chromatography (column: TSKgel ODS-80TM, carrier:ethanol/water/trifluoroacetic acid=5/95/0.04) and the speed at which the reaction filtrate was recovered was controlled to maintain the concentration of the reaction filtrate at 10% (w/w). The concentration of 2-amino-4-methylthiobutyronitrile contained in the reaction filtrate increased gradually, reached 0.5% (w/w) at 12 h after the initiation of the reaction, and the concentration was maintained for 20 days, during which the production speed of methionine was 5.03 g/h.

### Example 4. (Recovery of solid DL-methionine)

250 g of the reaction solution with bacteria separated therefrom (containing 25 g of methionine, 1.25 g of 2-amino-4-methylthiobutyronitrile, and 2.3% of ammonia) was set in a 500 mL-flask with a stirrer, and ammonia was distilled out under reduced pressure without heating with the use of a vacuum pump. Deposited Methionine obtained by distilling out ammonia was filtered and separated, and 11.3 g of methionine was obtained. 13.7 g of methionine and 1.25 g of 2-amino-4-methylthiobutyronitrile were contained in the mother liquor, and no degradation product was formed. The bacterial reaction-crystallization was performed repeatedly by using the mother liquor, while the recovery of obtained methionine was quantitative such as 99%, having purity of 99% or more, and no coloration was observed.

### Example 5. (Continuous production of DL-methionine by immobilized IFO 15564 strain.)

### (Culture and immobilization of IFO 15564 strain)

Two ml of medium containing 1.0% of tryptone, 0.5% of yeast extract, and 1.0% of sodium chloride, was poured into a test tube and sterilized for 20 min at 121° C. After 1 platinum loop of Rhodococcus rhodochrous IFO 15564 strain was inoculated into the test tube, the mixture was cultured with shaking overnight at 30°C, to prepare the pre-culture. Subsequently, 20 ml of medium of pH 7.2 (adjusted with 2N of caustic soda) containing 2.0% of corn steep liquor (filtered and sterilized), 1.0% of sucrose (sterilized for 20 min at 121° C), 0.5% of ε-caprolactam (sterilized for 20 min at 121°C), was poured into a 100 ml-Erlenmyer flask with a baffle reinoculated with 0.2 ml of the above-described pre-culture, and further cultured with shaking for 3 days at 30°C. Obtained culture solution of Rhodococcus rhodochrous IFO 15564 strain was subjected to centrifugation, then washed with ion-exchanged water, and the resultant was suspended in 1% (w/w) of sodium alginate solution so that the concentration as dried bacteria becomes 10% (w/w) . Then, the suspension was dropped to 0.1 M of calcium chloride solution to prepare immobilized bacteria-beads.

### (Continuous production of DL-methionine)

A column having an inside diameter of 30 mm was filled with 225 g of obtained bacteria-beads, and the column was equilibrate by flowing 1 1 of aqueous solution containing 10% (w/w) DL-methionine, 2.28% (w/w) ammonia, and 10 mM of calcium chloride at a flow late of 0.21/h. Then, the beads were poured into a 500 ml-three-necked flask incubated at 35°C, the equilibrated aqueous solution was added so that the total amount became 300 g, and then 2-amino-4-methylthiobutanamide was sequentially added to the resultant by stirring at a speed of 11.54g/h. On the other hand, the reaction solution was recovered through a suction filter at a speed of about 105 g/h so that the immobilized beads were not sucked out. At that time, 1.14% (w/w) of aqueous ammonia solution containing 10 mM of calcium chloride in equal amount of the reaction filtrate, was continuously replenished by using a pump coupled with a liquid level-sensor, so that the liquid level in the reaction container did not reduce. The concentration of DL-methionine of recovered reaction filtrate was measured every hour by using high performance liquid chromatography (column: TSKgel ODS-80TM, carrier: acetonitrile/water/trifluoroacetic acid=50/950/1) and the speed at which the reaction filtrate was recovered was controlled to maintain the concentration of the reaction filtrate at 10% (w/w). The continuous reaction was thus performed for 14 days. The production speed of methionine was 10.57 g/h on average.

### Example 6. (Recovery of solid DL-methionine)

250 g of the reaction filtrate obtained in Example 5 was sampled and set in a 500 mL-flask with a stirrer, and ammonia was distilled out under reduced pressure with the use of a vacuum pump. Deposited methionine obtained by distilling out ammonia was filtered and separated, and 11. 3 g of methionine was obtained. 13.7 g of methionine and 1. 02 g of 2-amino-4-methylthiobutanamide were contained in the mother liquor, and no degradation product formed. The bacterial reaction-crystallization was performed repeatedly by using the mother liquor, while the recovery of obtained methionine was quantitative such as 99%, having purity of 99% or more, and no coloration was observed.

### Industrial Applicability

According to the present invention, solid methioine required as a product form can be produced efficiently and easily as follows: by producing methionine dissolved in an aqueous ammonia solution, by using 2-amino-4-methylthiobutyronitrile, 2-amino-4-methylthiobutanamide or the like as raw material, and a biocatalyst having nitrile-hydrolyzing activity, amide-hydrolyzing activity or the like, and distilling out ammonia out of the resultant. In addition, the present invention can reduce energy-cost/amount of waste generation markedly in comparison with conventional chemical method for producing methionine.

## Claims

1. A method for producing methionine comprising the following steps: (1) a first step of converting raw material capable of producing methionine through hydrolysis into a form of methionine-containing aqueous ammonia solution through hydrolysis in an aqueous ammonia solution with a biocatalyst having hydrolyzing activity, (2) a second step of separating the biocatalyst from the methionine-containing aqueous ammonia solution obtained in the first step, and (3) a third step of distilling ammonia out of the methionine-containing aqueous ammonia solution separated in the second step to deposit and separate crystals of methionine.

2. The method for producing methionine according to claim 1, wherein 2-amino-4-methylthiobutyronitrile as raw material is hydrolyzed in an aqueous ammonia solution with a biocatalyst having nitrile-hydrolyzing activity.

3. The method for producing methionine according to claim 1, wherein 2-amino-4-methylthiobutanamide as raw material is hydrolyzed in an aqueous ammonia solution with a biocatalyst having amide-hydrolyzing activity.

4. The method for producing methionine according to any one of claims 1 to 3, wherein an aqueous solution containing 1.5 to 10 fold equivalent amount of ammonia corresponding to that of methionine in the methionine-containing aqueous ammonia solution obtained in the first step, is used as aqueous ammonia solution.

5. The method for producing methionine according to any one of claims 1 to 4, wherein the concentration of methionine in the methionine-containing aqueous ammonia solution obtained in the first step is 5-30 wt%.

6. The method for producing methionine according to any one of claims 1 to 5, wherein the biocatalyst is reused.

7. The method for producing methionine according to any one of claims 1 to 6, wherein an immobilized bacterium is used as the biocatalyst.

8. The method for producing methionine according to any one of claims 1 to 7 , wherein the distilled ammonia and the mother liquor from which crystals of methionine are separated and recovered are reused for hydrolysis reaction.

9. The method for producing methionine according to any one of claims 1 to 8, wherein the first step is performed under pressurization.
